# EUROPEAN PATENT APPLICATION

(11) **EP 3 287 165 A1**
(43) Date of publication of application: **28.02.2018**
(21) Application number: 16185984.8
(22) Date of filing: 26.08.2016
(51) Int. Cl.: A61M 16/00

(54) **MECHANICAL VENTILATION DEVICE**

(71) Applicant: Medec Benelux NV, 9300 Aalst (BE)
(72) Inventor: Braem, Kristof, 9300 Aalst (BE)
(74) Representative: LC Patents

(57) **Abstract**

The present invention is related to the field of anesthesia and perioperative mechanical ventilation of patients. It provides an improved protocol of perioperative mechanical ventilation of patients as well as a mechanical ventilation device performing said procedure.

The protocol of the instant application is a volume-controlled, time-cycled ventilation mode wherein a variable TV is applied at a constant respiration rate with a variable inspiratory time (I) and thus a variable I:E ratio, but wherein the variability in TV is such that the total volume delivered over time is kept constant, and wherein the variability in I is such that the peak level of inspiratory flow is kept at a constant level.

## Description

### FIELD OF THE INVENTION

The present invention is related to the field of anesthesia and perioperative mechanical ventilation of patients. It provides an improved protocol of perioperative mechanical ventilation of patients as well as a mechanical ventilation device performing said procedure.

### BACKGROUND TO THE INVENTION

In case of an operation the transition from awake, spontaneous breathing to the anesthetized state with spontaneous breathing in the supine position and the further transition to being paralyzed and mechanically ventilated introduce profound physiologic changes. With induction of anesthesia and mechanical ventilation in the supine position, patients quickly develop areas of dependent atelectasis with proportional right-to-left shunting. Anesthesia and mechanical ventilation also result in reduction in functional residual capacity to a point approaching or below closing capacity. The resulting airway closure may contribute to the dependent atelectasis, low ventilation/perfusion (V˙A/Q˙) ratios, and possibly some of the compliance changes seen during anesthesia. The overall result is an impairment of gas exchange with potential hypoxemia during anesthesia with mechanical ventilation. Patients undergoing abdominal surgery with placement of mechanical retractors and abdominal packs seem to be particularly prone to these complications.

Modern mechanical ventilators operating in control mode deliver respiratory rate (RR) and tidal volume (TV) in a monotonously regular manner. In contrast, normal ventilation is characterized by a variable or "noisy" signal. Most if not all physiologic signals manifest variability of a specific type. When transformed and displayed graphically, such signals plot as a straight line that mathematically describes a simple power law. Variable or noisy time sequences are associated with health, and their loss often heralds premorbid deterioration. The importance of variability in the respiratory pattern is partially explained by the anatomical structure of the lung. The airways and the pulmonary circulation are branching trees with a typical fractal structure in the sense that lower airway generations closely resemble higher generations, and small branches of the pulmonary circulation are similar to larger ones. Such a fractal structure maximizes the area for gas exchange and supports irregular gas-mixing in the lower branches. Breath-by-breath variation in tidal volume and respiratory rate contribute to sustain fast state transition, while minimizing the ratio between tissue stress and strain.

Thus where changes to a more monotonous signal during perioperative mechanical ventilation may result in the aforementioned complications, it is to be expected that the use of a physiological variability in the respiratory pattern, as observed in the healthy resting state, may be beneficial to improve function and reduce damage in the diseased lung. Studies (Mutch WA, Eschun GM, Kowalski SE, Graham MR, Girling LG, Lefevre GR. Biologically variable ventilation prevents deterioration of gas exchange during prolonged anaesthesia. Br J Anaesth. 2000;84:197-203) using a porcine model ventilated with variable RR and TV, a mode termed *biologically variable ventilation,* have presented experimental evidence supporting this contention, and there is independent confirmation from Arold *et al.* (Arold SP, Mora R, Lutchen KR, Ingenito EP, Suki B: Variable tidal volume ventilation improves lung mechanics and gas exchange in a rodent model of acute lung injury. Am J Respir Crit Care Med 2002; 165:366-71) in a rodent model, specifically as it relates to this mode of ventilation.

Notwithstanding the proven beneficial effects of the aforementioned *biologically variable ventilation* protocol, there are currently no commercially available ventilators capable of performing biologically variable ventilation. It is accordingly an objective of the instant application to address this unmet need.

### SUMMARY OF THE INVENTION

The present invention can be summarized in the following numbered embodiments.
1. A method for the ventilation (respiration) of a subject, said method comprising; applying a variable tidal volume (TV) with a variable inspiratory time (I) to the subject at a given Respiration Rate (RR); but characterized in that;
   a. The variability in TV is such that the total volume delivered is kept constant over time (expressed differently; the variability in TV is such that the minute volume (MV) is kept constant); and
   b. The variability in I is such that the peak level of inspiratory flow is kept at a constant level.
   As a variability in inspiratory time (I) inevitably results in a variability in inspiratory/expiratory ratio, the aforementioned method can alternatively be formulated as a method for the ventilation (respiration) of a subject, said method comprising; applying a variable tidal volume (TV) with a variable inspiratory/expiratory ratio (I:E-ratio) to the subject at a given Respiration Rate (RR); but characterized in that;
   c. The variability in TV is such that the total volume delivered is kept constant over time (expressed differently; the variability in TV is such that the minute volume (MV) is kept constant); and
   d. The variability in I:E-ratio is such that the peak level of inspiratory flow is kept at a constant level.
2. The method according to claim 1, wherein the RR is synchronized with the subject's attempt to inhale.
3. The method according to claim 1, wherein the RR is variable
4. The method according to any one of claims 1 to 3, wherein the variability in TV is from about 1 - 1000 % of a desired tidal volume, in particular from about 5 - 500 % of a desired (given) tidal volume (TVreq), more in particular from about 10 - 250 % of a desired tidal volume (TVreq); even more in particular from about 50 - 200 % of a desired tidal volume (TVreq).
5. The method according to claim 4 wherein the desired tidal volume is from about 5 - 1600 ml/breath.
6. The method according to any one of claims 2 to 5, wherein the variability in I:E-ratio is from about 4:1 to 1:10; in particular from 2:1 - 1:10.
7. Use of the method according to any one of claims 1 to 6 in a ventilator / respirator device.
8. An apparatus for ventilation (respiration) of a subject, said apparatus applying a variable tidal volume (TV) with a variable inspiratory time (I) to the subject at a given respiration rate; but characterized in that;
   - The variability in TV is such that the total volume delivered is kept constant over time (expressed differently; the variability in TV is such that the minute volume (MV) is kept constant ); and
   - The variability in I is such that the peak level of inspiratory flow is kept at a constant level.
9. An apparatus for ventilation (respiration) of a subject, said apparatus applying a variable tidal volume (TV) with a variable I:E-ratio to the subject at a given respiration rate; but characterized in that;
   - The variability in TV is such that the total volume delivered is kept constant over time (expressed differently; the variability in TV is such that the minute volume (MV) is kept constant ); and
   - The variability in I:E-ratio is such that the peak level of inspiratory flow is kept at a constant level.

### BRIEF DESCRIPTION OF THE DRAWINGS

With specific reference now to the figures, it is stressed that the particulars shown are by way of example and for purposes of illustrative discussion of the different embodiments of the present invention only. They are presented in the cause of providing what is believed to be the most useful and readily description of the principles and conceptual aspects of the invention. In this regard no attempt is made to show structural details of the invention in more detail than is necessary for a fundamental understanding of the invention. The description taken with the drawings making apparent to those skilled in the art how the several forms of the invention may be embodied in practice.

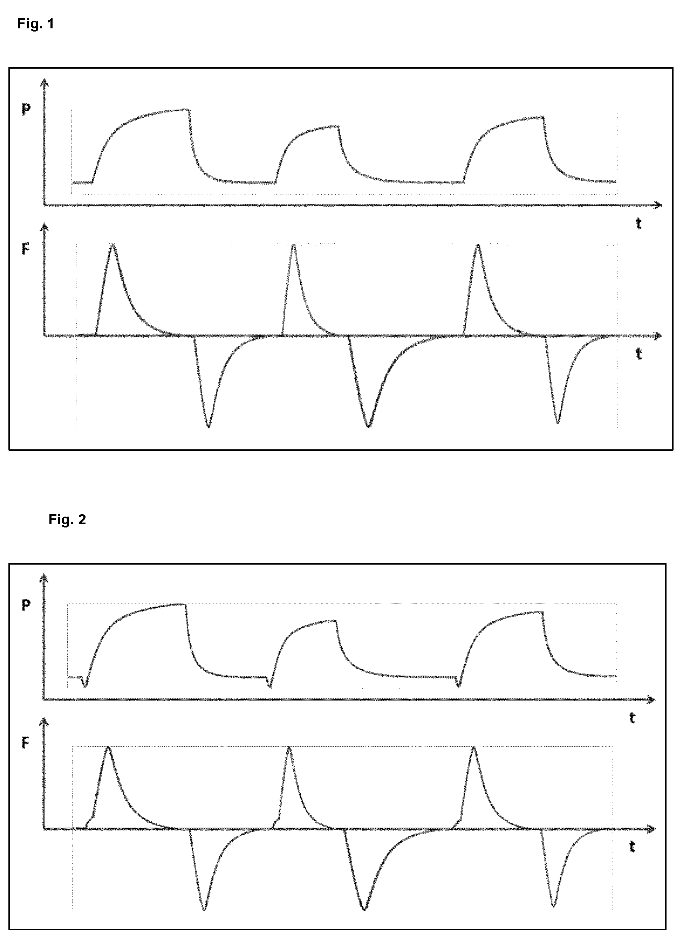

**Fig. 1****: Respiratory Pressure (cmH₂O) over time curve and Respiratory Flow (l/min) over time curve for the MVCV**
**Fig. 2****: Respiratory Pressure (cmH₂O) over time curve and Respiratory Flow (l/min) over time curve for the S-MVCV**

### DETAILED DESCRIPTION OF THE INVENTION

Compared to the *biologically variable ventilation* protocol, the protocol of the present invention generates variable ventilation by variability of parameters different from the parameters used in the *biologically variable ventilation* protocol. In the present invention, three parameters are controlled in the mechanical ventilator. As in the *biologically variable ventilation* protocol, also in the instant case the patient is ventilated with a variable tidal volume (TV), but where in the *biologically variable ventilation* protocol this is combined with a variability on the Respiration Rate (Frequency) (RR), this RR is kept constant in the procedure of the present invention. In addition, over time the protocol of the present invention maintains a constant minute volume, or expressed differently the total volume delivered is kept constant over time and it is delivered at a constant frequency, but during each ventilation cycle a variable tidal volume is being applied. Next to a variable TV with a constant minute volume, the protocol of the instant application further applies a variable inspiratory time and accordingly varies the inspiratory/expiratory ratio (I:E ratio). The inspiratory time is adjusted to maintain the peak inspiratory flow at a constant level. In practice the inspiratory time will be increased when a larger TV is being applied, thereby increasing the I:E ratio. Changing the I:E ratio in a ventilation protocol is against the teaching of recent studies which show that increasing the inspiratory time and I:E ratio aggravates VILI when higher tidal volumes are being used (Increasing the inspiratory time and I:E ratio during mechanical ventilation aggravates ventilator-induced lung injury in mice; Holger C Müller-Redetzky, Matthias Felten, Katharina Hellwig, Sandra-Maria Wienhold, Jan Naujoks, Bastian Opitz, Olivia Kershaw, Achim D Gruber, Norbert Suttorp, Martin Witzenrath; Crit Care. 2015; 19(1): 23. Published online 2015 Jan 28. doi: 10.1186/s13054-015-0759-2). Topping the I:E ratio by maintaining a constant peak inspiratory flow level, the protocol of the instant application evades the aforementioned problem and allows a more efficient gas exchange which is clinically beneficial to the patient.

To summarize, the protocol of the instant application is a volume-controlled, time-cycled ventilation mode wherein a variable TV is applied at a given (preset) respiration rate with a variable inspiratory time (I) and thus a variable I:E ratio, but wherein the variability in TV is such that the total volume delivered over time is kept constant (MV is constant), and wherein the variability in I is such that the peak level of inspiratory flow is kept at a constant level. Within this application, this protocol is being referred to as the *Multivariate Volume Controlled Ventilation (MVCV)* protocol. Knowledgeable of the existence synchronized mechanical ventilation devices, the protocol of the instant application can equally be performed in a synchronized form, wherein each respiration cycle is triggered with the patient's attempt to inhale. Still, even within this synchronizes mode, the total volume delivered over time is kept constant (MV is constant), and the peak level of inspiratory flow is kept at a constant level. Only the frequency is not a preset respiration rate, but synchronized with the patient's attempt to inhale. Within this application, this protocol is being referred to as the *Synchronized Multivariate Volume Controlled Ventilation (S-MVCV)* protocol.

As used herein, the tidal volume (TV) is the volume (ml) inhaled in a single breath, by setting a constant volume over time, i.e. the total volume (ml) inhaled per minute (Minute Volume MV), the range for the variability in TV will be influenced by the actual volume delivered in the past minute and the desired MV, and will be such that the total volume inhaled over time is kept constant at the preset respiration rate.

Where in a first aspect the variability in inspiratory time is such that the peak level of inspiratory flow is kept at a constant level, in another aspect of the present invention the variability in I is set by other elements. The method being based on a preset Respiration Rate (RR) the length of each respiratory cycle will be the same. By varying the inspiratory time, this inevitably influences the exhalation time, thus affecting the I:E ratio. The inspiratory time is varied in response to the variability of the tidal volume. I will increase during bigger breaths and decrease during smaller breaths, but the variability in I will be such that the length of each respiratory cycle is kept constant, as determined by the RR. In a particular embodiment the applied Inspiratory time (I) will not exceed 2/3 of the total breath duration.

It is accordingly an object of the present invention to provide in a first aspect a method for the ventilation (respiration) of a subject, said method comprising;
- Applying a variable tidal volume (TV) with a variable inspiratory time (I) to the subject at a constant respiration rate; but characterized in that;
   o The variability in TV is such that the total volume delivered is kept constant over time (expressed differently; the variability in TV is such that the minute volume (MV) is kept constant); and
   o The variability in I is such that the peak level of inspiratory flow is kept at a constant level

As the variability in inspiratory time (I) can equally be described by means of the inspiratory/expiratory ratio (I:E ratio); it is equally an object of the present invention to provide a method for the ventilation (respiration) of a subject, said method comprising;
- Applying a variable tidal volume (TV) with a variable I:E-ratio to the subject at a constant respiration rate; but characterized in that;
   o The variability in TV is such that the total volume delivered is kept constant over time (expressed differently; the variability in TV is such that the minute volume (MV) is kept constant); and
   o The variability in I:E-ratio is such that the peak level of inspiratory flow is kept at a constant level.

In one embodiment of the aforementioned methods the respiration rate is synchronized with the subject's attempt to inhale.

In one embodiment the variability in tidal volume in the aforementioned methods can be set with a degree of variability within the range of from about about 1 - 1000 % of a given tidal volume, in particular from about 5 - 500 % of a given (desired) tidal volume; more in particular from about 10 - 250 % of a given tidal volume; even more in particular from about 50 - 200 % of a given tidal volume. The given tidal volume (TVreq) set by the user and corresponding to the volume (ml) inhaled in a single breath, will be in the normal physiological range of a subject, for example in humans typically in the range of 5 - 1600 ml. Together with preset respiration rate the given tidal volume determines the Minute Volume MV, i.e. MV (inhalation ml/min) = TVreq (inhalation ml/breath) * RR (breaths/min)).

In a further embodiment the degree of variability in the aforementioned methods is limited depending on the set of tidal volume. For low TVreq the variability in TV will be in the range from about 5 - 200 % of TVreq; in particular in the range from about 25 - 150% of TVreq; even more in particular in the range from about 50 - 200% of TVreq; in another embodiment in the range from about 50 - 100% of TVreq. Low TVreq, are those typically used in protective lung ventilation, with for examples values of up to 800 ml / breath. For high TVreq the variability in TV will be in the range from about 50 - 500 % of TVreq; in particular in the range from about 75 - 250% of TVreq; even more in particular in the range from about 100 - 200% of TVreq. High TVreq are those used outside the values typically used in protective lung ventilation, with for examples values from 800 - 1600 ml/breath.

In a particular embodiment the variability in the TV is in accordance with the values presented in the below table.

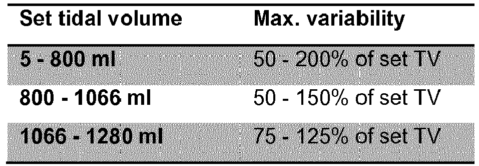

As explained hereinbefore, next to the variability in TV, the method of the instant application is characterized in a variable Inspiratory time (I), and accordingly in a variable I:E-ratio. In a first aspect the variability in I and I:E-ratio will be such that the peak level of inspiratory flow is kept at a constant level. However, in each instant and driven by the preset respiration rate, the length of each respiratory cycle has to be a constant value. Hence, the variability in I and the variability in the I:E ratio will be such that the length of each respiratory cycle is kept constant, in a particular embodiment the applied Inspiratory time (I) will not exceed 2/3 of the total breath duration. For the exemplary tidal volumes herein provided and typical respiration rates, this corresponds in a range of variability in the I:E-ratio from 4:1 to 1:10; in one embodiment the range of variability in I:E ratio in the aforementioned methods is from 2:1 - 1:10.

In another embodiment of the aforementioned methods the methods further comprise the application of a positive end-expiratory pressure (PEEP). It is within the skills of the anesthesist to determine the optimal PEEP value for a given subject. Low PEEP: 3 - 5 cmH2O is the typical physiological range used to preserve patient's normal Functional Residual Capacity (FRC). Moderate PEEP: 5 - 15 cmH2O, is the range typically used in case of refractory hypoxemia caused by ↑ intrapulmonary shunting with ↓ FRC and ↓ Compliance, and the most common range used for acute lung injury. High PEEP:> 15 cmH2O are the values used only for severe lung injury.

As evident from the foregoing, in the implementation of the *Multivariate Volume Controlled Ventilation (MVCV)* protocol, a desired tidal volume is (TVreq) and a desired Respiratory Rate (RR) is given, together this values determine the desired Minute Volume (MV), in ventilating the subject both the tidal volume and the inspiratory time will be varied per breathing cycle, but such that the minute volume is kept constant and such that the length of each respiratory cycle is kept constant. Any methodology seen fit to determine the allowable variability in tidal volume and in determining the allowable variability in inspiratory time for each breathing cycle, within the aforementioned boundaries is within the admit of the instant application. A sample approach to determine the variability in tidal volume and in inspiratory time for each breathing cycle is based on the following measurements and calculations.
- During the MVCV-protocol one measures the actual delivered tidal volume and the actual volume delivered in the past minute (MVeff)
- The difference between the desired Minute Volume (MV) and MVeff is calculated as a percentage (MVerr).
- Said percentage determines the variability range for the Tidal volume to be applied in the next respiration cycle.
- Within said range a random TV is selected (TVsel)
- Only if this TVsel is within the allowed range for the tidal volume parameter, this TVsel will be applied in the next respiration cycle else the closest TV is selected within the aforementioned variability range;
- The I:E-ratio is adjusted for the selected TV by multiplication of the inspiratory time (I) with the ratio of TVsel and TVreq, or alternatively the inspiratory time is adjusted (ladj) by multiplication of the inspiratory time (I) with the ratio of TVsel and TVreq. Within these calculations the reference value for the inspiratory time is the inspiratory time expected for the given TVreq and the given Respiratory Rate (RR),

The length of the inspiratory time (I) being adjusted in response to the variability in tidal volume, the overall procedure is controlled by the Minute Volume.

Optionally in the foregoing approach to determine the variability in tidal volume and in inspiratory time for each breathing cycle, the user may preset the variability range for the tidal volume. In said instance the preset variability range will be corrected with the calculated MVerr.

In a particular embodiment, eventually in combination with the preset variability range for the tidal volume, the adjusted inspiratory time is limited to 2/3 of the total breath duration.

Having developed this Multivariate Volume Controlled Ventilation protocol it is a further object of the present invention to provide the use of said protocol in a mechanical ventilator. As such the application further includes an apparatus (ventilator / respirator) for ventilation (respiration) of a subject, said apparatus applying a variable tidal volume (TV) with a variable inspiratory time (I) to the subject at a constant respiration rate; but characterized in that;
- The variability in TV is such that the total volume delivered is kept constant over time (expressed differently; the variability in TV is such that the minute volume is kept constant ); and
- The variability in I is such that the peak level of inspiratory flow is kept at a constant level.

As the variability in inspiratory time (I) can equally be described by means of the inspiratory/expiratory ratio (I:E ratio); it is equally an object of the present invention to provide an apparatus (ventilator / respirator) for ventilation (respiration) of a subject, said apparatus applying a variable tidal volume (TV) with a variable I:E-ratio to the subject at a constant respiration rate; but characterized in that;
- The variability in TV is such that the total volume delivered is kept constant over time (expressed differently; the variability in TV is such that the minute volume is kept constant); and
- The variability in I:E-ratio is such that the peak level of inspiratory flow is kept at a constant level.

The ventilator according to the invention will have an interface (such as a keyboard or touchscreen) to allow the user to set a desired tidal volume is (TVreq) and a desired Respiratory Rate (RR).

In performing the aforementioned protocol a ventilator according to the invention will comprise;
- Flow measurement means to measure the actual delivered tidal volume and the actual volume delivered in the past minute (MVeff)
- Processing means to determine the difference between the desired Minute Volume (MV) and MVeff, said difference being calculated as a percentage (MVerr).
- Processing means to determine the variability range for the Tidal volume to be applied in the next respiration cycle using the MVerr value obtained.
- Processing means to randomly select a TV (TVsel), within the thus obtained range
- Processing means to determine if this TVsel is within the allowed range for the tidal volume parameter. If within the allowed range this TVsel will be applied in the next respiration cycle else another TV is selected within the aforementioned variability range;
- Processing means to calculate ladj as the multiplication of the inspiratory time (I) with the ratio of TVsel and TVreq;
- Control means to apply TVsel and ladj in the next respiration cycle.

Optionally the ventilator may comprise a further user interface allowing the user to provide a variability range for the tidal volume *(supra).*

## Claims

1. A method for the ventilation (respiration) of a subject, said method comprising; applying a variable tidal volume (TV) with a variable inspiratory time (I) to the subject at a given Respiration Rate (RR); but **characterized in that**;
a. The variability in TV is such that the total volume delivered is kept constant over time (expressed differently; the variability in TV is such that the minute volume is kept constant); and
b. The variability in I is such that the peak level of inspiratory flow is kept at a constant level.

2. A method for the ventilation (respiration) of a subject, said method comprising; applying a variable tidal volume (TV) with a variable inspiratory/expiratory ratio (I:E-ratio) to the subject at a given Respiration Rate (RR); but **characterized in that**;
a. The variability in TV is such that the total volume delivered is kept constant over time (expressed differently; the variability in TV is such that the minute volume is kept constant); and
b. The variability in I:E-ratio is such that the peak level of inspiratory flow is kept at a constant level.

3. The method according to claim 1 or 2, wherein the RR is synchronized with the subject's attempt to inhale.

4. The method according to claim 1 or 2, wherein the RR is variable.

5. The method according to any one of claims 1 to 4, wherein the variability in TV is from about 1 - 1000 % of a desired tidal volume, in particular from about 5 - 500 % of a desired (given) tidal volume (TVreq), more in particular from about 10 - 250 % of a desired tidal volume (TVreq); even more in particular from about 50 - 200 % of a desired tidal volume (TVreq).

6. The method according to claim 5 wherein the desired tidal volume is from about 5 - 1600 ml/breath

7. The method according to any one of claims 2 to 6, wherein the variability in I:E-ratio is from about 4:1 to 1:10; in particular from 2:1 - 1:10.

8. Use of the method according to any one of claims 1 to 7 in a ventilator / respirator device.

9. An apparatus for ventilation (respiration) of a subject, said apparatus applying a variable tidal volume (TV) with a variable inspiratory time (I) to the subject at a constant respiration rate; but **characterized in that**;
- The variability in TV is such that the total volume delivered is kept constant over time (expressed differently; the variability in TV is such that the minute volume is kept constant); and
- The variability in I is such that the peak level of inspiratory flow is kept at a constant level.

10. An apparatus for ventilation (respiration) of a subject, said apparatus applying a variable tidal volume (TV) with a variable I:E-ratio to the subject at a constant respiration rate; but **characterized in that**;
- The variability in TV is such that the total volume delivered is kept constant over time (expressed differently; the variability in TV is such that the minute volume is kept constant); and
- The variability in I:E-ratio is such that the peak level of inspiratory flow is kept at a constant level.

11. An apparatus according to claims 9 or 10, comprising;
- Flow measurement means to measure the actual delivered tidal volume and the actual volume delivered in the past minute (MVeff)
- Processing means to determine the difference between the desired Minute Volume (MV) and MVeff, said difference being calculated as a percentage (MVerr).
- Processing means to determine the variability range for the Tidal volume to be applied in the next respiration cycle using the MVerr value obtained.
- Processing means to randomly select a TV (TVsel), within the thus obtained range
- Processing means to determine if this TVsel is within the allowed range for the tidal volume parameter. If within the allowed range this TVsel will be applied in the next respiration cycle else another TV is selected within the aforementioned variability range;
- Processing means to calculate ladj as the multiplication of the inspiratory time (I) with the ratio of TVsel and TVreq;
- Control means to apply TVsel and ladj in the next respiration cycle.

12. An apparatus according to any one of claims 9 to 11, comprising an interface (such as a keyboard or touchscreen) to allow the user to set a desired tidal volume is (TVreq) and a desired Respiratory Rate (RR).

13. An apparatus according to any one of claims 9 to 12, comprising an interface allowing the user to provide a variability range for the tidal volume.
